# EUROPEAN PATENT APPLICATION

(11) **EP 3 533 884 A1**
(43) Date of publication of application: **04.09.2019**
(21) Application number: 19151944.6
(22) Date of filing: 12.03.2014
(51) Int. Cl.: C12Q 1/70, C12P 19/34, A61K 35/12, A61K 35/66, C12Q 1/689, C12Q 1/6869

(54) **DNA SEQUENCES TO ASSESS CONTAMINATION IN DNA SEQUENCING**

(30) Priority: 15.03.2013 US 201361787451 P
(62) Divisional of application: 14767628.2
(71) Applicant: Ibis Biosciences, Inc., Carlsbad, CA 92008 (US)
(72) Inventor: ESHOO, Mark W., San Diego, CA California 92130 (US); MOTLEY, Stanley, Issaquah, WA 98027 - 4004 (US)
(74) Representative: Chapman, Desmond Mark

(57) **Abstract**

The present invention relates to systems and methods for detecting contamination in nucleic acid analyses. In particular, the present invention relates to systems and methods for detecting contamination in DNA sequencing assays.

SEQUENCE LISTING

<110>  IBIS BIOSCIENCES, INC.
       ESHOO, Mark W.
       MOTLEY, Stanley

<120>  DNA SEQUENCES TO ASSESS CONTAMINATION IN DNA SEQUENCING

<130>  33189WO1ORD

<140>  PCT/US2014/024516
<141>  2014-03-12

<150>  US 61/787,451
<151>  2013-03-15

<160>  17

<170>  PatentIn version 3.5

<210>  1
<211>  40
<212>  DNA
<213>  Homo sapiens

<400>  1
ccattaacct tccctctaca cttatcatct tcacaattct                              40


<210>  2
<211>  41
<212>  DNA
<213>  Homo sapiens

<400>  2
ccattaaacc ttccctctac acttatcatc ttcacaattc t                            41


<210>  3
<211>  37
<212>  DNA
<213>  Homo sapiens

<400>  3
ccattaacct tccctctatt atcatcttca caattct                                 37


<210>  4
<211>  39
<212>  DNA
<213>  Homo sapiens

<400>  4
cattaacttc cctctacact ttatcatctt cacaattct                               39


<210>  5
<211>  36
<212>  DNA
<213>  Homo sapiens

<400>  5
ccattaacct tctacactta tcatcttcac aattct                                  36

<210>  6
<211>  32
<212>  DNA
<213>  Homo sapiens

<400>  6
cttccctcta cacttatcat cttcacaatt ct                                    32


<210>  7
<211>  5
<212>  DNA
<213>  Homo sapiens

<400>  7
attct                                                                   5


<210>  8
<211>  40
<212>  DNA
<213>  Bacillus anthracis

<400>  8
caccttgacg gtacctaacc agaaagccac ggctaactac                            40


<210>  9
<211>  38
<212>  DNA
<213>  Bacillus anthracis

<400>  9
caccttgacg gtacctaacc agaaagccgg ctaactac                              38


<210>  10
<211>  37
<212>  DNA
<213>  Bacillus anthracis

<400>  10
caccttgacg gtacctaacc agaaagccac taactac                               37


<210>  11
<211>  39
<212>  DNA
<213>  Bacillus anthracis

<400>  11
caccttgacg gtacctaacc agaagccacg gctaactac                             39


<210>  12
<211>  39
<212>  DNA
<213>  Bacillus anthracis

<400>  12
caccttgacg gtacctacca gaaagccacg gctaactac                             39

<210> 13
<211> 38
<212> DNA
<213> Bacillus anthracis

<400> 13
caccttgacg gtacctaacc agaaagccac ggcactac 38


<210> 14
<211> 36
<212> DNA
<213> Bacillus anthracis

<400> 14
cacctgacgg tacctaacca gaccacggct aactac 36


<210> 15
<211> 39
<212> DNA
<213> Bacillus anthracis

<400> 15
caccttgacg gtacctaaca gaaagccacg gctaactac 39


<210> 16
<211> 37
<212> DNA
<213> Bacillus anthracis

<400> 16
cttgacggta cctaaccaga aagccacggc taactac 37


<210> 17
<211> 39
<212> DNA
<213> Bacillus anthracis

<400> 17
caccttgacg gtacctaacc agaaagccac ggctaacta 39

## Description

This application claims priority to Provisional Patent Application Serial No. 61/787,451, filed March 15, 2013, which is herein incorporated by reference in its entirety.

### STATEMENT REGARDING FEDERALLY SPONSORED RESEARCH OR DEVELOPMENT

This invention was made with government support under HSHQDC-11-C-00090 awarded by DHS. The government has certain rights in the invention.

### FIELD OF INVENTION

The present invention relates to systems and methods for detecting contamination in nucleic acid analyses. In particular, the present invention relates to systems and methods for detecting contamination in DNA sequencing assays.

### BACKGROUND

Because it is ultra-sensitive, next Generation DNA sequencing (NGS) is subject to contamination from a wide range of sources. Many NGS sequencing runs have detectable levels of contaminating sequences. The presence of contaminants can lead to false positive results when sequencing nucleic acids present at low levels. For example, if one is looking for a rare cancer variant from a clinical sample that is found at 1% of the sequences for a locus of interest, it can be difficult to determine if this is from the sample or contaminating human DNA.

Twenty percent of the finished Genebank genomes have been shown to contain contaminating human DNA sequences. Every step of the analysis is vulnerable to contamination (Mark S. Longo et al., Feb., 2011 PLoSONE; Otto Erlwein et al 2011 PLoS ONE 6(8)).

Methods to assess levels of contamination are needed to reduce false-positive results in sequencing-based diagnostic assays.

### SUMMARY

The present invention relates to systems and methods for detecting contamination in nucleic acid analyses. In particular, the present invention relates to systems and methods for detecting contamination in DNA sequencing assays.

For example, in some embodiments, the present invention provides a method of determining the presence, absence, or level of contaminating nucleic acid in a sample, comprising: a) sequencing a nucleic acid sample comprising a target nucleic acid and a control nucleic acid, wherein the control nucleic acid is from contaminating nucleic acid to generate a sequencing data set; b) determining the level of control nucleic acid and target nucleic acid in the sequencing data set; c) comparing the level of control nucleic acid to the level of target nucleic acid; and d) determining the presence, absence, or level of contaminating nucleic acid in the sample based on the comparing. In some embodiments, the contaminating nucleic acid is human and the control nucleic acid is human mitochondrial nucleic acid. In some embodiments, the contaminating nucleic acid is bacterial and the control nucleic acid is bacterial 16S nucleic acid (e.g., 16S nucleic acid is from *B. anthrasis*)*.* In some embodiments, determining the levels of control and target nucleic acids is performed using a computer processor and computer software. In some embodiments, a high level of control nucleic acid relative to target nucleic acid is indicative of a high level of contaminating nucleic acid in the sample. In some embodiments, the target nucleic acid is a human nucleic acid (e.g., a nucleic acid variant, deletion, or amplification). In some embodiments, the sample is a human sample and the target nucleic acid is viral, bacterial, or fungal. In some embodiments, the sequencing is next generation sequencing (e.g., utilizing techniques described herein). In some embodiments, the target and control nucleic acids are amplified prior to sequencing (e.g., using whole genome amplification).

In further embodiments, the present invention provides kits comprising, consisting essentially of, or consisting of, components necessary, useful, or sufficient for determining the presence, absence, or level of nucleic acid contamination in a sequencing assay. For example, in some embodiments, kits comprise primers, nucleic acid anchors for use in sequencing assays, probes, or reagents. In some embodiments, kits comprise software for determining the presence, absence, or level of nucleic acid contamination in a sequencing reaction.

In yet other embodiments, the present invention provides a reaction mixture comprising a nucleic acid sequencing target nucleic acid mixture; and a contaminating nucleic acid and at least one sequencing primer annealed to the contaminating nucleic acid.

Additional embodiments will be apparent to persons skilled in the relevant art based on the teachings contained herein.

### BRIEF DESCRIPTION OF THE DRAWINGS

Figure 1 shows the results of NGS using human mitochondrial sequence to detect the presence of human DNA.
Figure 2 shows that many alignments with 16S sequences to *B.anthrasis* 16S sequences were found in *B.cereus* data set.

### DETAILED DESCRIPTION

The present invention relates to systems and methods for detecting contamination in nucleic acid analyses. In particular, the present invention relates to systems and methods for detecting contamination in DNA sequencing assays.

### Definitions

To facilitate an understanding of the present technology, a number of terms and phrases are defined below. Additional definitions are set forth throughout the detailed description.

As used herein, "a" or "an" or "the" can mean one or more than one. For example, "a" widget can mean one widget or a plurality of widgets.

The term "label" as used herein refers to any atom or molecule that can be used to provide a detectable (preferably quantifiable) effect, and that can be attached to a nucleic acid or protein. Labels include but are not limited to dyes; radiolabels such as ³²P; binding moieties such as biotin; haptens such as digoxgenin; luminogenic, phosphorescent or fluorogenic moieties; and fluorescent dyes alone or in combination with moieties that can suppress or shift emission spectra by fluorescence resonance energy transfer (FRET). Labels may provide signals detectable by fluorescence, radioactivity, colorimetry, gravimetry, X-ray diffraction or absorption, magnetism, enzymatic activity, and the like. A label may be a charged moiety (positive or negative charge) or alternatively, may be charge neutral. Labels can include or consist of nucleic acid or protein sequence, so long as the sequence comprising the label is detectable.

The term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*e.g*., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer should be sufficiently long to prime the synthesis of extension products in the presence of the inducing agent. The exact lengths of the primers will depend on many factors, including temperature, source of primer and the use of the method.

The term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the terms "subject" and "patient" refer to any animal, such as a dog, a cat, a bird, livestock, and particularly a mammal, and preferably a human.

As used herein, the term "sensitivity" is defined as a statistical measure of performance of an assay (e.g., method, test), calculated by dividing the number of true positives by the sum of the true positives and the false negatives.

As used herein, the term "specificity" is defined as a statistical measure of performance of an assay (e.g., method, test), calculated by dividing the number of true negatives by the sum of true negatives and false positives.

As used herein, the term "amplicon" refers to a nucleic acid generated via amplification reaction. The amplicon is typically double stranded DNA; however, it may be RNA and/or DNA:RNA. The amplicon comprises DNA complementary to a sample nucleic acid. In some embodiments, primer pairs are configured to generate amplicons from a sample nucleic acid. As such, the base composition of any given amplicon may include the primer pair, the complement of the primer pair, and the region of a sample nucleic acid that was amplified to generate the amplicon. One skilled in the art understands that the incorporation of the designed primer pair sequences into an amplicon may replace the native sequences at the primer binding site, and complement thereof. In certain embodiments, after amplification of the target region using the primers the resultant amplicons having the primer sequences are used for subsequent analysis (e.g. base composition determination). In some embodiments, the amplicon further comprises a length that is compatible subsequent analysis.

The term "amplifying" or "amplification" in the context of nucleic acids refers to the production of multiple copies of a polynucleotide, or a portion of the polynucleotide, typically starting from a small amount of the polynucleotide (e.g., as few as a single polynucleotide molecule), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR) are forms of amplification. Amplification is not limited to the strict duplication of the starting molecule. For example, the generation of multiple cDNA molecules from a limited amount of RNA in a sample using reverse transcription (RT)-PCR is a form of amplification. Furthermore, the generation of multiple RNA molecules from a single DNA molecule during the process of transcription is also a form of amplification.

As used herein, the term "anchor" refers to a nucleic acid that serves as a template for annealing of a second nucleic acid. In some embodiments, anchors serve as annealing sites for sequencing primers (e.g., in next generation sequencing methods).

As used herein, the term "sample" is used in its broadest sense. In one sense, it is meant to include a representative portion or culture obtained from any source, including biological and environmental sources. Biological samples may be obtained from animals (including humans) and encompass fluids, solids, tissues, and gases. Biological samples include blood products, such as plasma, serum, and the like. Environmental samples include environmental material such as surface matter, soil, mud, sludge, biofilms, water, and industrial samples. Such examples are not however to be construed as limiting the sample types applicable to the present invention.

### Embodiments of the technology

Although the disclosure herein refers to certain illustrated embodiments, it is to be understood that these embodiments are presented by way of example and not by way of limitation.

As described above, sequencing methods (e.g., next generation sequencing methods) provide high sensitivity. However, the high level of sensitivity can result in detection of contaminating human or microbial nucleic acids being detected as false positive results in diagnostic or screening assays.

Accordingly, embodiments of the present invention provide systems and methods that utilize conserved housekeeping sequences (e.g., mitochondrial and ribosomal DNA sequences) to determine the levels of nucleic acid contamination in sequencing or other nucleic acid detection reactions (e.g., NGS).

In some embodiments, the presence of human mitochondrial sequences is used to detect contaminating human sequences in a sample. For example, in some embodiments, NGS methods and systems utilize primers or anchors to specifically amplify and sequence human mitochondrial sequences. In some embodiments, whole genome sequencing and/or amplification methods are utilized and the number of reads attributable to human mitochondrial sequences are identified using bioinformatics systems and methods.

In some embodiments, the presence of bacterial 16S sequences is used to detect contaminating bacterial nucleic acids in a sample. For example, in some embodiments, NGS methods and systems utilize primers to specifically amplify and sequence bacterial 16S sequences. In some embodiments, whole genome sequencing and/or amplification methods are utilized and the number of reads attributable to bacterial 16S sequences are identified using bioinformatics systems and methods. In some embodiments, the bacterial 16S sequence is a 16S sequence from *B.anthrasis.*

In some embodiments, the level of nucleic acid attributable to the housekeeping nucleic acid (e.g., as determined by the number of sequencing reads) is compared to the level of a target nucleic acid (e.g., as determined by the number of sequencing reads). The amount of control nucleic acid compared to the amount of target nucleic acid sequenced is used to determine the presence or level of contamination.

In some embodiments, bioinformatics methods are utilized to determine the level of control (e.g., human mitochondrial or bacterial 16S) nucleic acids in a sample. The levels are then compared to levels of target nucleic acids in the sample to determine the level of contamination. The level of contamination present is then used to determine if assay results (e.g., presence or absence of a given target sequence) are true positive results, due to contamination, or suspicious.

### I. Amplification

In some embodiments, prior to, following or concurrently with sequencing, nucleic acids are amplified. In some embodiments, amplification methods are whole genome amplification methods.

Illustrative non-limiting examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (*e.g*., PCR) require that RNA be reversed transcribed to DNA prior to amplification (*e.g*., RT-PCR), whereas other amplification techniques directly amplify RNA (*e.g*., TMA and NASBA).

The polymerase chain reaction (U.S. Pat. Nos. 4,683,195, 4,683,202, 4,800,159 and 4,965,188, each of which is herein incorporated by reference in its entirety), commonly referred to as PCR, uses multiple cycles of denaturation, annealing of primer pairs to opposite strands, and primer extension to exponentially increase copy numbers of a target nucleic acid sequence. In a variation called RT-PCR, reverse transcriptase (RT) is used to make a complementary DNA (cDNA) from mRNA, and the cDNA is then amplified by PCR to produce multiple copies of DNA. For other various permutations of PCR *see, e.g*., U.S. Pat. Nos. 4,683,195, 4,683,202 and 4,800,159; Mullis et al., Meth. Enzymol. 155: 335 (1987); and, Murakawa et al., DNA 7: 287 (1988), each of which is herein incorporated by reference in its entirety.

Transcription mediated amplification (U.S. Pat. Nos. 5,480,784 and 5,399,491, each of which is herein incorporated by reference in its entirety), commonly referred to as TMA, synthesizes multiple copies of a target nucleic acid sequence autocatalytically under conditions of substantially constant temperature, ionic strength, and pH in which multiple RNA copies of the target sequence autocatalytically generate additional copies. *See, e.g*., U.S. Pat. Nos. 5,399,491 and 5,824,518, each of which is herein incorporated by reference in its entirety. In a variation described in U.S. Publ. No. 20060046265 (herein incorporated by reference in its entirety), TMA optionally incorporates the use of blocking moieties, terminating moieties, and other modifying moieties to improve TMA process sensitivity and accuracy.

The ligase chain reaction (Weiss, R., Science 254: 1292 (1991), herein incorporated by reference in its entirety), commonly referred to as LCR, uses two sets of complementary DNA oligonucleotides that hybridize to adjacent regions of the target nucleic acid. The DNA oligonucleotides are covalently linked by a DNA ligase in repeated cycles of thermal denaturation, hybridization and ligation to produce a detectable double-stranded ligated oligonucleotide product.

Strand displacement amplification (Walker, G. et al., Proc. Natl. Acad. Sci. USA 89: 392-396 (1992); U.S. Pat. Nos. 5,270,184 and 5,455,166, each of which is herein incorporated by reference in its entirety), commonly referred to as SDA, uses cycles of annealing pairs of primer sequences to opposite strands of a target sequence, primer extension in the presence of a dNTPaS to produce a duplex hemiphosphorothioated primer extension product, endonuclease-mediated nicking of a hemimodified restriction endonuclease recognition site, and polymerase-mediated primer extension from the 3' end of the nick to displace an existing strand and produce a strand for the next round of primer annealing, nicking and strand displacement, resulting in geometric amplification of product. Thermophilic SDA (tSDA) uses thermophilic endonucleases and polymerases at higher temperatures in essentially the same method (EP Pat. No. 0 684 315).

Other amplification methods include, for example: nucleic acid sequence based amplification (U.S. Pat. No. 5,130,238, herein incorporated by reference in its entirety), commonly referred to as NASBA; one that uses an RNA replicase to amplify the probe molecule itself (Lizardi et al., BioTechnol. 6: 1197 (1988), herein incorporated by reference in its entirety), commonly referred to as Qβ replicase; a transcription based amplification method (Kwoh et al., Proc. Natl. Acad. Sci. USA 86:1173 (1989)); and, self-sustained sequence replication (Guatelli et al., Proc. Natl. Acad. Sci. USA 87: 1874 (1990), each of which is herein incorporated by reference in its entirety). For further discussion of known amplification methods *see* Persing, David H., "In Vitro Nucleic Acid Amplification Techniques" in Diagnostic Medical Microbiology: Principles and Applications (Persing et al., Eds.), pp. 51-87 (American Society for Microbiology, Washington, DC (1993)).

In some embodiments, amplification is isothermal amplification method. In some embodiments, amplification methods are solid-phase amplification, polony amplification, colony amplification, emulsion PCR, bead RCA, surface RCA, surface SDA, etc., as will be recognized by one of skill in the art. In some embodiments, amplification methods that results in amplification of free DNA molecules in solution or tethered to a suitable matrix by only one end of the DNA molecule are used. In some embodiments, methods that rely on bridge PCR, where both PCR primers are attached to a surface (see, e.g., WO 2000/018957, U.S. 7,972,820; 7,790,418 and Adessi et al., Nucleic Acids Research (2000): 28(20): E87; each of which are herein incorporated by reference) are used. In some cases the methods of the invention can create a "polymerase colony technology", or "polony", referring to a multiplex amplification that maintains spatial clustering of identical amplicons (see Harvard Molecular Technology Group and Lipper Center for Computational Genetics website). These include, for example, in situ polonies (Mitra and Church, Nucleic Acid Research 27, e34, Dec. 15, 1999), in situ rolling circle amplification (RCA) (Lizardi et al., Nature Genetics 19, 225, July 1998), bridge PCR (U.S. Pat. No. 5,641,658), picotiter PCR (Leamon et al., Electrophoresis 24, 3769, November 2003), and emulsion PCR (Dressman et al., PNAS 100, 8817, Jul. 22, 2003).

In some embodiments, whole genome amplification is utilized. The first whole genome amplification methods were described in 1992, and were based on the principles of the polymerase chain reaction. Zhang and coworkers (Zhang, L., et al. Proc. Natl. Acad. Sci. USA, 1992, 89: 5847-5851; herein incorporated by reference) developed the primer extension PCR technique (PEP) and Telenius and collaborators (Telenius et al., Genomics. 1992, 13(3):718-25; herein incorporated by reference) designed the degenerate oligonucleotide-primed PCR method (DOP-PCR). PEP involves a high number of PCR cycles, generally using Taq polymerase and 15 base random primers that anneal at a low stringency temperature. DOP-PCR is a method which generally uses Taq polymerase and semi-degenerate oligonucleotides that bind at a low annealing temperature at approximately one million sites within the human genome. The first cycles are followed by a large number of cycles with a higher annealing temperature, allowing only for the amplification of the fragments that were tagged in the first step.

Multiple displacement amplification (MDA, also known as strand displacement amplification; SDA) is a non-PCR-based isothermal method based on the annealing of random hexamers to denatured DNA, followed by strand-displacement synthesis at constant temperature (Blanco et al., 1989, J. Biol. Chem. 264:8935-40; Dean, F.B. et al. (2002) Comprehensive human genome amplification using multiple displacement amplification; Proc. Natl. Acad. Sci. USA 99,5261; and Van, J. et al. (2004) Assessment of multiple displacement amplification in molecular epidemiology. Biotechniques 37, 136; all of which are herein incorporated by reference). It has been applied to small genomic DNA samples, leading to the synthesis of high molecular weight DNA with limited sequence representation bias (Lizardi et al., Nature Genetics 1998, 19, 225-232; Dean et al., Proc. Natl. Acad. Sci. U.S.A. 2002, 99, 5261-5266; both of which are herein incorporated by reference). As DNA is synthesized by strand displacement, a gradually increasing number of priming events occur, forming a network of hyper-branched DNA structures. The reaction can be catalyzed by the Phi29 DNA polymerase or by the large fragment of the Bst DNA polymerase. The Phi29 DNA polymerase possesses a proofreading activity resulting in error rates 100 times lower than the Taq polymerase.

Examples of nucleic acid polymerases suitable for use in embodiments of the present invention include, but are not limited to, DNA polymerase (Klenow fragment, T4 DNA polymerase), thermostable DNA polymerases (Perler F. B. et al., Adv. Protein Chem. 1996, 48:377-435) identified and cloned in a variety of thermostable bacteria (such as Taq, VENT, Pfu, Tfl DNA polymerases) as well as their genetically modified derivatives (TaqGold, VENTexo, Pfu exo). Preferably the nucleic acid polymerase used for colony primer extension is stable under temperature at which the primer and template hybridization results specific enough to avoid incomplete or spurious amplifications of the template.

The amplification solution contains preferably, as nucleotide precursors, deoxyribonucleotide triphosphates, for example dATP, dTTP, dCTP, dGTP, naturally or non-naturally occurring, for example modified with a fluorescent or radioactive group. A large variety of synthetically modified nucleic acids have been developed for chemical and biological methods in order to increase the detectability and/or the functional diversity of nucleic acids. These functionalized/modified molecules can be fully compatible with natural polymerizing enzymes, maintaining the base pairing and replication properties of the natural counterparts, as recently reviewed (Thum O et al., Angew. Chem. Int. Ed. 2001, 40 (21): 3990-3993).

Other components of the amplification solution are added consequently to the choice of the nucleic acid polymerase, and they are essentially corresponding to compounds known in the art as being effective to support the activity of each polymerase. The concentration of compounds like dimethyl sulfoxide (DMSO), Bovine Serum Albumin (BSA), Triton X-100, or MgCl₂ is well known in the prior art as being important to have an optimal amplification, and therefore the operator can easily adjust such concentrations for the methods of the present invention on the basis of the examples presented hereafter.

### II. Sequencing

In some embodiments, nucleic acid sequencing methods are utilized for detection. In some embodiments, the technology provided herein finds use in a Second Generation (a.k.a. Next Generation or Next-Gen), Third Generation (a.k.a. Next-Next-Gen), or Fourth Generation (a.k.a. N3-Gen) sequencing technology including, but not limited to, pyrosequencing, sequencing-by-ligation, single molecule sequencing, sequence-by-synthesis (SBS), massive parallel clonal, massive parallel single molecule SBS, massive parallel single molecule real-time, massive parallel single molecule real-time nanopore technology, etc. Morozova and Marra provide a review of some such technologies in Genomics, 92: 255 (2008), herein incorporated by reference in its entirety. Those of ordinary skill in the art will recognize that because RNA is less stable in the cell and more prone to nuclease attack experimentally RNA is usually reverse transcribed to DNA before sequencing.

A number of DNA sequencing techniques are known in the art, including fluorescence-based sequencing methodologies (See, e.g., Birren et al., Genome Analysis: Analyzing DNA, 1, Cold Spring Harbor, N.Y.; herein incorporated by reference in its entirety). In some embodiments, the technology finds use in automated sequencing techniques understood in that art. In some embodiments, the present technology finds use in parallel sequencing of partitioned amplicons (PCT Publication No: WO2006084132 to Kevin McKernan et al., herein incorporated by reference in its entirety). In some embodiments, the technology finds use in DNA sequencing by parallel oligonucleotide extension (See, e.g., U.S. Pat. No. 5,750,341 to Macevicz et al., and U.S. Pat. No. 6,306,597 to Macevicz et al., both of which are herein incorporated by reference in their entireties).

Additional examples of sequencing techniques in which the technology finds use include the Church polony technology (Mitra et al., 2003, Analytical Biochemistry 320, 55-65; Shendure et al., 2005 Science 309, 1728-1732; U.S. Pat. No. 6,432,360, U.S. Pat. No. 6,485,944, U.S. Pat. No. 6,511,803; herein incorporated by reference in their entireties), the 454 picotiter pyrosequencing technology (Margulies et al., 2005 Nature 437, 376-380; US 20050130173; herein incorporated by reference in their entireties), the Solexa single base addition technology (Bennett et al., 2005, Pharmacogenomics, 6, 373-382; U.S. Pat. No. 6,787,308; U.S. Pat. No. 6,833,246; herein incorporated by reference in their entireties), the Lynx massively parallel signature sequencing technology (Brenner et al. (2000). Nat. Biotechnol. 18:630-634; U.S. Pat. No. 5,695,934; U.S. Pat. No. 5,714,330; herein incorporated by reference in their entireties), and the Adessi PCR colony technology (Adessi et al. (2000). Nucleic Acid Res. 28, E87; WO 00018957; herein incorporated by reference in its entirety).

Next-generation sequencing (NGS) methods share the common feature of massively parallel, high-throughput strategies, with the goal of lower costs in comparison to older sequencing methods (see, e.g., Voelkerding et al., Clinical Chem., 55: 641-658, 2009; MacLean et al., Nature Rev. Microbiol., 7: 287-296; each herein incorporated by reference in their entirety). NGS methods can be broadly divided into those that typically use template amplification and those that do not. Amplification-requiring methods include pyrosequencing commercialized by Roche as the 454 technology platforms (e.g., GS 20 and GS FLX), the Solexa platform commercialized by Illumina, and the Supported Oligonucleotide Ligation and Detection (SOLiD) platform commercialized by Applied Biosystems. Non-amplification approaches, also known as single-molecule sequencing, are exemplified by the HeliScope platform commercialized by Helicos BioSciences, and emerging platforms commercialized by VisiGen, Oxford Nanopore Technologies Ltd., Life Technologies/Ion Torrent, and Pacific Biosciences, respectively.

In pyrosequencing (Voelkerding et al., Clinical Chem., 55: 641-658, 2009; MacLean et al., Nature Rev. Microbiol., 7: 287-296; U.S. Pat. No. 6,210,891; U.S. Pat. No. 6,258,568; each herein incorporated by reference in its entirety), template DNA is fragmented, end-repaired, ligated to adaptors, and clonally amplified in-situ by capturing single template molecules with beads bearing oligonucleotides complementary to the adaptors. Each bead bearing a single template type is compartmentalized into a water-in-oil microvesicle, and the template is clonally amplified using a technique referred to as emulsion PCR. The emulsion is disrupted after amplification and beads are deposited into individual wells of a picotitre plate functioning as a flow cell during the sequencing reactions. Ordered, iterative introduction of each of the four dNTP reagents occurs in the flow cell in the presence of sequencing enzymes and luminescent reporter such as luciferase. In the event that an appropriate dNTP is added to the 3' end of the sequencing primer, the resulting production of ATP causes a burst of luminescence within the well, which is recorded using a CCD camera. It is possible to achieve read lengths greater than or equal to 400 bases, and 10⁶ sequence reads can be achieved, resulting in up to 500 million base pairs (Mb) of sequence.

In the Solexa/Illumina platform (Voelkerding et al., Clinical Chem., 55: 641-658, 2009; MacLean et al., Nature Rev. Microbiol., 7: 287-296; U.S. Pat. No. 6,833,246; U.S. Pat. No. 7,115,400; U.S. Pat. No. 6,969,488; each herein incorporated by reference in its entirety), sequencing data are produced in the form of shorter-length reads. In this method, single-stranded fragmented DNA is end-repaired to generate 5'-phosphorylated blunt ends, followed by Klenow-mediated addition of a single A base to the 3' end of the fragments. A-addition facilitates addition of T-overhang adaptor oligonucleotides, which are subsequently used to capture the template-adaptor molecules on the surface of a flow cell that is studded with oligonucleotide anchors. The anchor is used as a PCR primer, but because of the length of the template and its proximity to other nearby anchor oligonucleotides, extension by PCR results in the "arching over" of the molecule to hybridize with an adjacent anchor oligonucleotide to form a bridge structure on the surface of the flow cell. These loops of DNA are denatured and cleaved. Forward strands are then sequenced with reversible dye terminators. The sequence of incorporated nucleotides is determined by detection of post-incorporation fluorescence, with each fluor and block removed prior to the next cycle of dNTP addition. Sequence read length ranges from 36 nucleotides to over 50 nucleotides, with overall output exceeding 1 billion nucleotide pairs per analytical run.

Sequencing nucleic acid molecules using SOLiD technology (Voelkerding et al., Clinical Chem., 55: 641-658, 2009; MacLean et al., Nature Rev. Microbiol., 7: 287-296; U.S. Pat. No. 5,912,148; U.S. Pat. No. 6,130,073; each herein incorporated by reference in their entirety) also involves fragmentation of the template, ligation to oligonucleotide adaptors, attachment to beads, and clonal amplification by emulsion PCR. Following this, beads bearing template are immobilized on a derivatized surface of a glass flow-cell, and a primer complementary to the adaptor oligonucleotide is annealed. However, rather than utilizing this primer for 3' extension, it is instead used to provide a 5' phosphate group for ligation to interrogation probes containing two probe-specific bases followed by 6 degenerate bases and one of four fluorescent labels. In the SOLiD system, interrogation probes have 16 possible combinations of the two bases at the 3' end of each probe, and one of four fluors at the 5' end. Fluor color, and thus identity of each probe, corresponds to specified color-space coding schemes. Multiple rounds (usually 7) of probe annealing, ligation, and fluor detection are followed by denaturation, and then a second round of sequencing using a primer that is offset by one base relative to the initial primer. In this manner, the template sequence can be computationally re-constructed, and template bases are interrogated twice, resulting in increased accuracy. Sequence read length averages 35 nucleotides, and overall output exceeds 4 billion bases per sequencing run.

In certain embodiments, the technology finds use in nanopore sequencing (see, e.g., Astier et al., J. Am. Chem. Soc. 2006 Feb 8; 128(5):1705-10, herein incorporated by reference). The theory behind nanopore sequencing has to do with what occurs when a nanopore is immersed in a conducting fluid and a potential (voltage) is applied across it. Under these conditions a slight electric current due to conduction of ions through the nanopore can be observed, and the amount of current is exceedingly sensitive to the size of the nanopore. As each base of a nucleic acid passes through the nanopore, this causes a change in the magnitude of the current through the nanopore that is distinct for each of the four bases, thereby allowing the sequence of the DNA molecule to be determined.

In certain embodiments, the technology finds use in HeliScope by Helicos BioSciences (Voelkerding et al., Clinical Chem., 55: 641-658, 2009; MacLean et al., Nature Rev. Microbiol., 7: 287-296; U.S. Pat. No. 7,169,560; U.S. Pat. No. 7,282,337; U.S. Pat. No. 7,482,120; U.S. Pat. No. 7,501,245; U.S. Pat. No. 6,818,395; U.S. Pat. No. 6,911,345; U.S. Pat. No. 7,501,245; each herein incorporated by reference in their entirety). Template DNA is fragmented and polyadenylated at the 3' end, with the final adenosine bearing a fluorescent label. Denatured polyadenylated template fragments are ligated to poly(dT) oligonucleotides on the surface of a flow cell. Initial physical locations of captured template molecules are recorded by a CCD camera, and then label is cleaved and washed away. Sequencing is achieved by addition of polymerase and serial addition of fluorescently-labeled dNTP reagents. Incorporation events result in fluor signal corresponding to the dNTP, and signal is captured by a CCD camera before each round of dNTP addition. Sequence read length ranges from 25-50 nucleotides, with overall output exceeding 1 billion nucleotide pairs per analytical run.

The Ion Torrent technology is a method of DNA sequencing based on the detection of hydrogen ions that are released during the polymerization of DNA (see, e.g., Science 327(5970): 1190 (2010); U.S. Pat. Appl. Pub. Nos. 20090026082, 20090127589, 20100301398, 20100197507, 20100188073, and 20100137143, incorporated by reference in their entireties for all purposes). A microwell contains a template DNA strand to be sequenced. Beneath the layer of microwells is a hypersensitive ISFET ion sensor. All layers are contained within a CMOS semiconductor chip, similar to that used in the electronics industry. When a dNTP is incorporated into the growing complementary strand a hydrogen ion is released, which triggers a hypersensitive ion sensor. If homopolymer repeats are present in the template sequence, multiple dNTP molecules will be incorporated in a single cycle. This leads to a corresponding number of released hydrogens and a proportionally higher electronic signal. This technology differs from other sequencing technologies in that no modified nucleotides or optics are used. The per-base accuracy of the Ion Torrent sequencer is ∼99.6% for 50 base reads, with ∼100 Mb generated per run. The read-length is 100 base pairs. The accuracy for homopolymer repeats of 5 repeats in length is ∼98%. The benefits of ion semiconductor sequencing are rapid sequencing speed and low upfront and operating costs.

The technology finds use in another nucleic acid sequencing approach developed by Stratos Genomics, Inc. and involves the use of Xpandomers. This sequencing process typically includes providing a daughter strand produced by a template-directed synthesis. The daughter strand generally includes a plurality of subunits coupled in a sequence corresponding to a contiguous nucleotide sequence of all or a portion of a target nucleic acid in which the individual subunits comprise a tether, at least one probe or nucleobase residue, and at least one selectively cleavable bond. The selectively cleavable bond(s) is/are cleaved to yield an Xpandomer of a length longer than the plurality of the subunits of the daughter strand. The Xpandomer typically includes the tethers and reporter elements for parsing genetic information in a sequence corresponding to the contiguous nucleotide sequence of all or a portion of the target nucleic acid. Reporter elements of the Xpandomer are then detected. Additional details relating to Xpandomer-based approaches are described in, for example, U.S. Pat. Pub No. 20090035777, entitled "High Throughput Nucleic Acid Sequencing by Expansion," filed June 19, 2008, which is incorporated herein in its entirety.

Other emerging single molecule sequencing methods include real-time sequencing by synthesis using a VisiGen platform (Voelkerding et al., Clinical Chem., 55: 641-58, 2009; U.S. Pat. No. 7,329,492; U.S. Pat. App. Ser. No. 11/671956; U.S. Pat. App. Ser. No. 11/781166; each herein incorporated by reference in their entirety) in which immobilized, primed DNA template is subjected to strand extension using a fluorescently-modified polymerase and florescent acceptor molecules, resulting in detectible fluorescence resonance energy transfer (FRET) upon nucleotide addition.

### III. Data Analysis

In some embodiments, a computer-based analysis program is used to translate the raw data generated by the detection assay (*e.g*., level of contamination human or microbial nucleic acids) into data of predictive value for an end user (e.g., medical personnel). The user can access the predictive data using any suitable means. Thus, in some preferred embodiments, the present invention provides the further benefit that the user, who is not likely to be trained in genetics or molecular biology, need not understand the raw data. The data is presented directly to the end user in its most useful form. The user is then able to immediately utilize the information in order to determine useful information (e.g., in medical diagnostics, research, or screening).

The present invention contemplates any method capable of receiving, processing, and transmitting the information to and from laboratories conducting the assays, information provides, medical personal, and subjects. For example, in some embodiments of the present invention, a sample (*e.g*., blood, cell or tissue sample) is obtained from a subject and submitted to a profiling service (*e.g*., lab at a medical facility, genomic profiling business, etc.), located in any part of the world (*e.g*., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g*., a cheek swab) and directly send it to a profiling center. Where the sample comprises previously determined biological information, the information may be directly sent to the profiling service by the subject (*e.g*., an information card containing the information may be scanned by a computer and the data transmitted to a computer of the profiling center using an electronic communication systems). Once received by the profiling service, the sample is processed and a profile is produced (*e.g*., level of contamination), specific for the information desired for the subject.

The data is then prepared in a format suitable for interpretation by the end user (e.g., medical personnel). For example, rather than providing raw data, the prepared format may represent a conclusion or assessment (*e.g*., level of contamination nucleic acids; presence of microbial nucleic acid or variant human nucleic acid, etc.) for the subject. The data may be displayed to the user by any suitable method. For example, in some embodiments, the profiling service generates a report that can be printed for the user or displayed on a computer monitor.

In some embodiments, the information is first analyzed at a local medical facility or at a regional facility. The raw data is then sent to a central processing facility for further analysis and/or to convert the raw data to information useful for the user. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the end user, the subject, or researchers.

### IV. Kits and Systems

In some embodiments, the present invention provides kits and systems for the amplification and/or analysis of nucleic acids. In some embodiments, kits include reagents necessary, sufficient or useful for analysis and detection of contaminating nucleic acid sequences (e.g., primers, probes, anchors, solid supports, reagents, controls, instructions, etc.). For example, in some embodiments, kits comprise primers and anchors for amplification and sequencing of samples (e.g., potentially contaminated sample). In some embodiments, kits include analysis software (e.g., to analyze sequencing data and determine the level of contaminating nucleic acids).

In some embodiments, kits comprise one or more containers that comprise reagents, primers, probes, anchors, solid supports, buffers, and the like. In some embodiments, each component of the kit is packaged in a separate container. In some embodiments, the containers are packed and/or shipped in the same kit or box for use together. In some embodiments, one or more components of the kit are shipped and/or packaged separately.

In some embodiments, systems include automated sample and reagent handling devices (e.g., robotics).

### EXPERIMENTAL

The following examples serve to illustrate certain preferred embodiments and aspects of the present invention and are not to be construed as limiting the scope thereof.

### Example 1

### Human mitochondrial sequences as marker for contamination

NextGENe (NGS Software) was used to look for human mitochondrial sequences in data sets, which may contain human DNA.

The test data set is from depleted human serum sample subject to WGA and NGS that was positive for herpes.

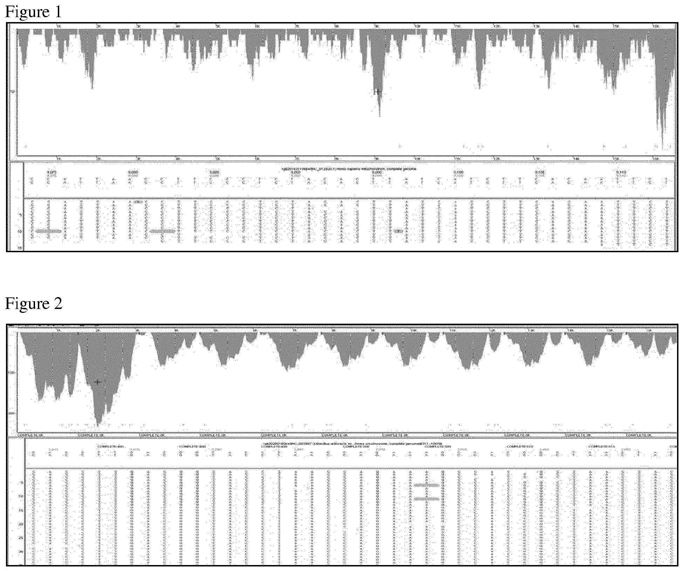

Figure 1 shows the results of NGS using human mitochondrial sequence to detect presence of human DNA. Many alignments with human mitochondrial sequences were found in the data set from depleted human serum sample that was positive for herpes.

### Bacterial 16S Sequences as Marker for Contamination

NextGENe (NGS Software) was used to look for bacterial 16S sequences in data sets, which may contain unknown bacterial DNA contamination.

NGS data set from 10 fg of *Bacillus cereus* DNA was subjected to WGA and NGS. Eleven different 16S sequences from *B.anthrasis* were used to detect contaminating bacterial sequences.

Results are shown in Figure 2. Figure 2 shows that many alignments with 16S sequences to *B.anthrasis* 16S sequences were found in *B.cereus* data set.

### The invention provides the following numbered embodiments:

1. A method of detecting contaminating nucleic acid in a sample, comprising:
   a) sequencing a nucleic acid sample comprising a target nucleic acid and a control nucleic acid, wherein said control nucleic acid is from said contaminating nucleic acid to generate a sequencing data set;
   b) determining the level of said control nucleic acid and said target nucleic acid in said sequencing data set;
   c) comparing the level of said control nucleic acid to the level of said target nucleic acid; and
   d) determining the presence, absence, or level of contaminating nucleic acid in said sample based on said comparing.
2. The method of embodiment 1, wherein said contaminating nucleic acid is human and said control nucleic acid is human mitochondrial nucleic acid.
3. The method of embodiment 1, wherein said contaminating nucleic acid is bacterial and said control nucleic acid is bacterial 16S nucleic acid.
4. The method of any one of embodiments 1 to 3, wherein said determining the levels of said control and target nucleic acids is performed using a computer processor and computer software.
5. The method of embodiment 1, wherein a high level of control nucleic acid relative to target nucleic acid is indicative of a high level of contaminating nucleic acid in said sample.
6. The method of embodiment 1, wherein said target nucleic acid is a human nucleic acid.
7. The method of embodiment 8, wherein said target nucleic acid is a nucleic acid variant, deletion, or amplification.
8. The method of embodiment 1, wherein said sample is a human sample and said target nucleic acid is viral, bacterial, or fungal.
9. The method of any one of embodiments 1 to 8, wherein said sequencing is next generation sequencing.
10. The method of any one of embodiments 1 to 9, wherein said target and control nucleic acids are amplified prior to sequencing.
11. The method of embodiment 10, wherein said amplifying comprises whole genome amplification.

All publications and patents mentioned in the above specification are herein incorporated by reference in their entirety for all purposes. Various modifications and variations of the described compositions, methods, and uses of the technology will be apparent to those skilled in the art without departing from the scope and spirit of the technology as described. Although the technology has been described in connection with specific exemplary embodiments, it should be understood that the invention as claimed should not be unduly limited to such specific embodiments. Indeed, various modifications of the described modes for carrying out the invention that are obvious to those skilled in the relevant fields are intended to be within the scope of the following claims.

## Claims

1. A system for detecting contaminating nucleic acid in a sample, wherein said system is configured to:
a) generate a sequencing data set by sequencing a nucleic acid sample comprising a target nucleic acid and a control nucleic acid, wherein said control nucleic acid is from said contaminating nucleic acid;
b) determine the level of said control nucleic acid and said target nucleic acid in said sequencing data set;
c) compare the level of said control nucleic acid to the level of said target nucleic acid; and
d) determine the presence, absence, or level of contaminating nucleic acid in said sample based on said comparing.

2. The system of claim 1, wherein said contaminating nucleic acid is human and said control nucleic acid is human mitochondrial nucleic acid, and wherein optionally the system comprises primers to specifically amplify and sequence human mitochondrial sequences

3. The system of claim 1, wherein said contaminating nucleic acid is bacterial and said control nucleic acid is bacterial 16S nucleic acid, and wherein optionally the system comprises primers to specifically amplify and sequence bacterial 16S sequences.

4. The system of any one of claims 1 to 3, wherein the system comprises a computer processor and computer software for determining the levels of said control and target nucleic acids.

5. The system of claim 1, wherein said target nucleic acid is a human nucleic acid.

6. The system of claim 5, wherein said target nucleic acid is a nucleic acid variant, deletion, or amplification.

7. The system of claim 1, wherein said sample is a human sample and said target nucleic acid is viral, bacterial, or fungal.

8. The system of any one of claims 1 to 8, wherein said sequencing is next generation sequencing.

9. The system of any one of claims 1 to 8, further configured to amplify said target and control nucleic acids prior to sequencing.

10. The system of claim 9, wherein the system is configured for whole genome amplification.

11. A method of detecting contaminating nucleic acid in a sample, comprising:
a) sequencing a nucleic acid sample comprising a target nucleic acid and a control nucleic acid, wherein said control nucleic acid is from said contaminating nucleic acid to generate a sequencing data set;
b) determining the level of said control nucleic acid and said target nucleic acid in said sequencing data set;
c) comparing the level of said control nucleic acid to the level of said target nucleic acid; and
d) determining the presence, absence, or level of contaminating nucleic acid in said sample based on said comparing.

12. The method of claim 11, wherein said contaminating nucleic acid is human and said control nucleic acid is human mitochondrial nucleic acid.

13. The method of claim 11, wherein said contaminating nucleic acid is bacterial and said control nucleic acid is bacterial 16S nucleic acid.

14. The method of any one of claims 11 to 13, wherein said determining the levels of said control and target nucleic acids is performed using a computer processor and computer software.

15. A kit for detecting contaminating nucleic acid in a sample according to the method of any of claims 11-14, comprising:
(a) primers and anchors for amplification and sequencing of samples; and
(b) analysis software to analyze sequencing data and determine the level of contaminating nucleic acids.
